# EUROPEAN PATENT SPECIFICATION

(11) **EP 0 346 929 B1**
(45) Date of publication and mention of the grant of the patent: **27.04.1994**
(21) Application number: 89111000.9
(22) Date of filing: 16.06.1989
(51) Int. Cl.: A61K 31/415, A61K 31/425, A61K 31/44

(54) **Treating agent for Raynaud's disease**
Mittel zur Behandlung von Raynaud's Krankheit
Agent pour le traitement de la maladie de Raynaud

(30) Priority: 17.06.1988 JP 149445/88
(43) Date of publication of application: 20.12.1989
(73) Proprietor: DAIICHI PHARMACEUTICAL CO., LTD., Chuo-ku, Tokyo 103 (JP)
(72) Inventor: Ashida, Shinichiro c/o Daiichi Seiyaku Research, Tokyo (JP); Sakuma, Kyoko c/o Daiichi Seiyaku Research, Tokyo (JP)
(74) Representative: Kinzebach, Werner, Dr.

## Description

The present invention relates to the use of at least one compound of formula (I): 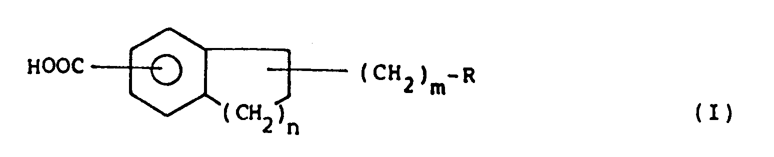
wherein R represents an imidazolyl group; n represents 1 or 2; and m represents an integer of from 1 to 4,
or a salt thereof for preparing a pharmaceutical composition for preventing and/or treating Raynaud's Disease.

The compounds represented by formula (I) are known as treating agent for ischemic heart diseases as disclosed in U.S. Patent Nos. 4,665,188 and 4,777,257 but unknown for their activity in prevention and treatment of peripheral circulatory disturbances.

In Patent Abstracts, volume 11, no. 13, (C-397) [24 60], January 14, 1987 it is disclosed that inter alia compounds of the formula(I)as defined above have excellent activity to suppress the synthesis of thromboxane A2. Said compounds are therfore used for the treatment of thrombosis and cerebrovascular disease.

In Thromb.Res. vol. 45, no. 4, February 15, 1987, 393-393-401 experiments are disclosed which relate to the urinary excretion of the major urinary metabolites of thromboxane B2 and prostacyclin in patients with confirmed deep vein thrombosis. By measurements of these major urinary metabolites it is possible to monitor the in vivo formation of thromboxane A2. From the results can be concluded that thromboxane A2 is involved in the course of events associated with deep vein thrombosis.

In Thromb.Res. vol. 44, no. 2, October 15, 1986, a study is described concerning thromboxane systhesis in platelets from patients with severe atherosclerosis. For this purpose, the thrombin stimulated thromboxane synthesis was determined in patients with peripheral vascular disease. The results indicate an increased synthesis of thromboxane.

It has now surprisingly been found that the compounds of formula (I) exhibit excellent effects on Raynaud's disease.

The present invention therefore relates to the use of the compounds of formula (I) for preparing a pharmaceutical composition for treating and/or preventing Raynaud's disease.

The salts of the compounds of formula (I) which can be used in the present invention include acid addition salts formed with inorganic acids (e.g., hydrochloric acid, sulfuric acid, and nitric acid) or organic acids (e.g.. fumaric acid, tartaric acid, maleic acid, succinic acid, and oxalic acid); and alkali metal salts or alkaline earth metal salts formed from the carboxyl group and alkali metals (e.g., sodium and potassium) or alkaline earth metals (e.g.. calcium and magnesium).

The compounds of formula (I) and salts thereof were tested for acute toxicity (LD₅₀) in rats (p.o.) and, as a result, proved highly safe.

The compounds of formula (I) and their salts can be formulated in various pharmaceutical preparations, such as tablets, powders, capsules, injectable solutions, and the like, by known pharmaceutical techniques. The compounds of formula (I) or salts thereof are usually administered orally, subcutaneously, intramuscularly, or intravenously.

The dose level of the compounds of formula (I) or salts thereof usually ranges from about 100 to about 1,000 mg/day/adult (body weight: about 60 kg) in oral administration.

As demonstrated in Test Example hereinafter given, the compounds of formula (I) and salts thereof exhibited excellent effects to suppress lesions in a peripheral arterial occlusive disease model, a typical disease model of peripheral circulatory disturbances. The compounds of formula (I) and the salts thereof are therefore excellent as preventing and treating agents for Raynaud's disease.

The present invention is now illustrated in greater detail by way of the following Test Examples.

### TEST EXAMPLE 1

### Effect on Progression of Laurate-Induced Peripheral Arterial Occlusive Diseases in Rats

Male Wister-Imamichi rats weighing from 370 to 422 g (purchased from Dobutsu Hanshoku Kenkyukai) were used as test animals.

Rats were anesthetized with pentobarbital (50 mg/kg, i.p.), and the right femoral artery was exposed by surgical incision. 0.1 mℓ of a sodium laurate solution (1 mg of lauric acid/mℓ-saline, pH=8.0) was injected to the right femoral artery, haemostasis was secured by application of a surgical binding "Alonalpha" (a trade name, produced by Toa Gosei Kagaku Co.) to the punctured site of the artery, and the incision was closed with the surgical binding. The animals were then fed ad lib.

The animals were observed for progression of the lesions. The degree of the lesions was graded to 0 - IV, 4 days after the operation for gangrene and 6 days after the operation for mummification or falling off as follows: normal apperance, 0; the affected region was limited to the nail parts, I; to the fingers, II; to the whole paw, III; and extended to the lower leg, IV.

As a test compound, 6-(1-imidazolylmethyl)-5,6,7,8-tetrahydronaphthalene-2-carboxylic acid hydrochloride 1/2 hydrate (hereinafter referred to as Compound A) was dissolved in physiological saline and administered to the left femoral vein at a dose of 3 mg/5 mℓ/kg 5 minutes before the laurate injection to the femoral artery. The effect of the Compound A on suppression of the lesions were examined. The animals in control group were injected with 5 mℓ/kg of saline alone in place of the Compound A. The results obtained are shown in Table 1 below.

**TABLE 1**

| | Number of Animals Suffering From: | | | | | | | | | |
|---|---|---|---|---|---|---|---|---|---|---|
| | Gangrene After 4 Days | | | | | Mummification or Falling Off After 6 Days | | | | |
| | 0 | I | II | III | IV | 0 | I | II | III | IV |
| Control Group | | 2 | 1 | | 4 | | 1 | 2 | | 4 |
| Treated Group | 2 | 3 | 2 | | | 2 | 1 | 3 | 1 | |

As is apparent from Table 1, gangrene was seen in the affected paw and the lower leg of the control group 4 days after the injection of the sodium laurate solution, and the gangrened part mummified or fell off 6 days after the laurate injection. To the contrary, intravenous injection of 3 mg/kg of the Compound A reduced the grade of lesions.

Accordingly, it was confirmed that the Compound A is useful for the prevention and treatment of Raynaud's disease.

### TEST EXAMPLE 2

Acute toxicity of the Compound A in rats (p.o.) is shown in Table 2.

**TABLE 2**

| Acute Toxicity (rat, p.o.) LD₅₀ (mg/kg) | |
|---|---|
| Male | Female |
| 2438 | 1994 |

## Claims

1. The use of at least one compound of formula (I): 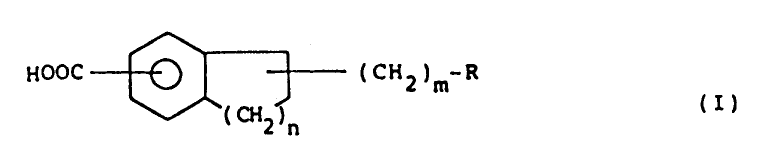 wherein R represents an imidazolyl group; n represents 1 or 2; and m represents an integer of from 1 to 4,
or a salt thereof for preparing a pharmaceutical composition for preventing and/or treating Raynaud's disease.

2. The use according to claim 1, wherein the compound of formula (I) is 6-(1-imidazolylmethyl)-5,6,7,8,-tetrahydronaphthalene-2-carboxylic acid of the formula: 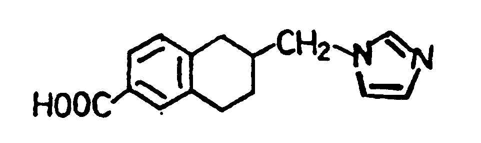 or a salt thereof.

3. The use according to claim 2, wherein the compound of formula (I) is 6-(1-imidazolylmethyl)-5,6,7,8,-tetrahydronaphthalene-2-carboxylic acid hydrochloride hemihydrate of the formula:

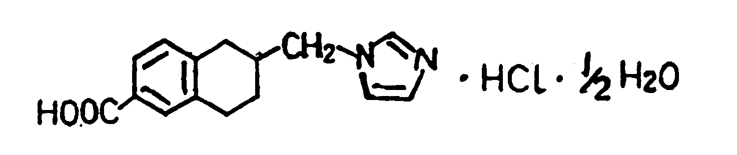

## Patentansprüche

1. Verwendung wenigstens einer Verbindung der allgemeinen Formel (I): 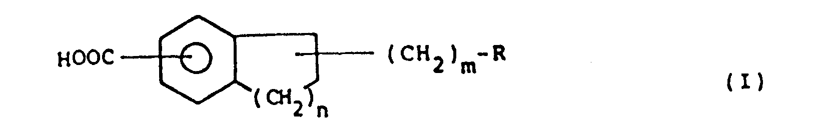 worin R für eine Imidazolylgruppe steht; n für 1 oder 2 steht; und m für eine ganze Zahl von 1 bis 4 steht;
oder eines Salzes davon zur Herstellung eines pharmazeutischen Mittels zur Prävention und/oder Behandlung der Raynaud-Krankheit.

2. Verwendung nach Anspruch 1, worin die Verbindung der Formel (I) 6-(1-Imidazolylmethyl)-5,6,7,8,-tetrahydronaphthalin-2-carbonsäure der Formel: 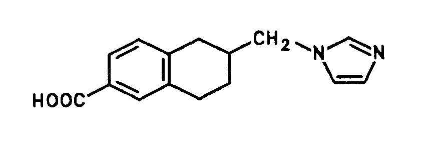 oder ein Salz davon ist.

3. Verwendung nach Anspruch 2, worin die Verbindung der Formel (I) 6-(1-Imidazolylmethyl)-5,6,7,8,-tetrahydronaphthalin-2-carbonsäure-hydochlorid-hemihydrat der Formel: 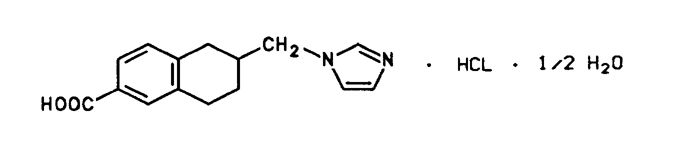 ist.

## Revendications

1. Utilisation d'au moins un compose de formule (I): 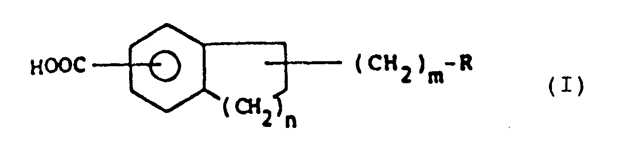 dans laquelle R représente un groupe imidazolyle; n représente 1 ou 2; et m représente un entier de 1 à 4, ou un sel de celui-ci, pour la préparation d'une composition pharmaceutique pour prévenir et/ou traiter la maladie de Raynaud.

2. Utilisation selon la revendication 1, dans laquelle le composé de formule (I) est l'acide 6-(1-imidazolylméthyl)-5,6,7,8-tétrahydronaphtalène-2-carboxylique de formule: 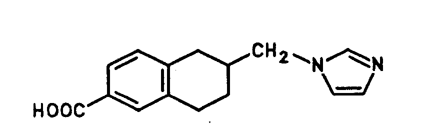 ou un sel de celui-ci.

3. Utilisation selon la revendication 2, dans laquelle le composé de formule (I) est l' hémihydrate du chlorhydrate de l'acide 6-(1-imidazolylméthyl)-5,6,7,8-tétrahydronaphtalène-2-carboxylique de formule:
